# EUROPEAN PATENT APPLICATION

(11) **EP 3 610 863 A1**
(43) Date of publication of application: **19.02.2020**
(21) Application number: 18776267.9
(22) Date of filing: 28.03.2018
(51) Int. Cl.: A61K 31/047, A61K 8/34, A61K 8/60, A61K 31/7004, A61P 17/00, A61Q 19/00

(54) **AGENT FOR ENHANCING SKIN BARRIER, MEDICINAL COMPOSITION, MEDICATED COSMETIC AND BEAUTY CARE METHOD**

(30) Priority: 31.03.2017 JP 2017071379
(71) Applicant: CAC Corporation, Nagareyama-shi, Chiba 270-0114 (JP)
(72) Inventor: YAMADA Kiyoko, Nagareyama-shi Chiba 270-0114 (JP); MATSUSHITA Kenji, Nagoya-shi Aichi 468-0022 (JP)
(74) Representative: Potter Clarkson
(86) International application number: PCT/JP2018/013013
(87) International publication number: WO 2018/181599

(57) **Abstract**

An agent for enhancing the skin barrier that can enhance skin barrier functions is provided. The agent for enhancing the skin barrier, including at least one of a monosaccharide and a sugar alcohol, in which a pH is 4.0 to 7.0 is provided.

## Description

### [Technical Field]

The present invention relates to an agent for enhancing the skin barrier, a medicinal composition, a medicated cosmetic, and a beauty care method.

Priority is claimed on Japanese Patent Application No. 2017-071379, filed March 31, 2017, the content of which is incorporated herein by reference.

### [Background Art]

The epidermis has a moisturizing function of preventing the evaporation of water in the body and retaining the water content of the skin, and a skin barrier function of preventing the invasion of Acari, dust, pollen dust, microorganisms, and the like.

In symptoms of inflammation which is seen in various skin diseases, atopic dermatitis, psoriasis, contact dermatitis, and the like, significant loss of water from the skin is able to be recognized. The loss of water involves a deterioration in moisturizing functions and a deterioration in skin barrier functions.

It has been reported that as skin barrier functions deteriorate, sensitization due to allergens, bacteria, and the like through the skin increases, and accordingly, allergic reactions, skin inflammation, and the like are induced. For example, in elderly people, symptoms such as dermatitis and pressure ulcers are easily aggravated when the skin barrier functions deteriorate and skin barrier functions are not sufficiently restored. Due to such facts, it is expected that there will be development of agents such as an agent for enhancing the skin barrier which can promote enhancement of skin barrier functions.

Based on the above-described circumstances, Patent Literature 1 discloses that glucose promotes HMGB1 production, induces extracellular release of HMGB1, enhances proliferation and migration of normal cells, and thereby promotes tissue repair.

### [Citation List]

### [Patent Literature]

[Patent Literature 1]
Japanese Unexamined Patent Application, First Publication No. 2013-147480

### [Summary of Invention]

### [Technical Problem]

However, an agent for enhancing the skin barrier that can further enhance skin barrier functions is in demand.

An object of the present invention is to provide an agent for enhancing the skin barrier that can further enhance skin barrier functions.

### [Solution to Problem]

Filaggrin is a protein present in cells that form the stratum granulosum in the epidermis and is a protein that retains the water content of the skin and thereby contributes to a moisturizing function. Claudin-1 is a membrane protein that constitutes tight junctions that connect cells in the epidermis. Claudin-1 is a protein that prevents invasion of allergens, microorganisms, and the like into cells, and contributes to a function of preventing water evaporation from the body surface.

As a result of intensive studies on an agent for enhancing the skin barrier which promotes skin tissue repair, the inventors of the present invention have found that a liquid, which contains at least one of a monosaccharide and a sugar alcohol and has a pH in an acidic region, has an effect of promoting the production of Filaggrin and Claudin-1 which are proteins relating to the skin barrier. The inventors of the present invention have presumed that if the production of these proteins relating to the skin barrier is promoted, skin barrier functions can be enhanced, and therefore have completed the present invention.

That is, the present invention has the following aspects [1] to [6].
[1] An agent for enhancing the skin barrier, including at least one of a monosaccharide and a sugar alcohol, in which a pH is 4.0 to 7.0.
[2] The agent for enhancing the skin barrier according to [1], in which glucose is contained as the monosaccharide.
[3] The agent for enhancing the skin barrier according to [1], in which mannitol is contained as the sugar alcohol.
[4] The agent for enhancing the skin barrier according to any one of [1] to [3], in which the pH is 5.0 to 7.0.
[5] A medicinal composition including the agent for enhancing the skin barrier according to any one of [1] to [4], in which a pH is 4.0 to 7.0.
[6] A medicated cosmetic including the agent for enhancing the skin barrier according to any one of [1] to [4], in which a pH is 4.0 to 7.0.
[7] The medicated cosmetic according to [6], which is in a liquid form, a gel form, or a sheet form.
[8] A beauty care method including a step of applying the medicated cosmetic according to [6] or [7] to skin or a mucous membrane.

### [Advantageous Effects of Invention]

According to the present invention, it is possible to provide an agent for enhancing the skin barrier that can further enhance skin barrier functions.

### [Brief Description of Drawings]

Fig. 1 is a graph showing evaluation results of an expression level of a Filaggrin protein of Examples 1 to 6 and Comparative Examples 1 to 4.
Fig. 2 is a graph showing evaluation results of an mRNA expression level of Filaggrin of Examples 2, 4, and 6 and Comparative Examples 2 and 4.
Fig. 3 is a graph showing evaluation results of an mRNA expression level of Claudin-1 of Examples 2, 4, and 6 and Comparative Examples 2 and 4.
Fig. 4 is a graph showing evaluation results of an expression level of a Filaggrin protein of Example 7 and Comparative Example 7.
Fig. 5 is a graph showing evaluation results of a cytotoxicity percentage of Examples 1 to 6 and Comparative Examples 1 to 4.
Fig. 6 shows photographs of the appearance of a mouse pinna evaluated by a mouse model with atopic dermatitis.
Fig. 7 shows photographs of results of staining tissue sections of the mouse pinna evaluated by the mouse model with atopic dermatitis.
Fig. 8 is a graph showing evaluation results of mRNA expression levels of Filaggrin and Claudin-1 of Reference Test 1.
Fig. 9 is a graph showing evaluation results of an expression level of a Filaggrin protein of Reference Test 2.
Fig. 10 is a graph showing evaluation results of an expression level of a Claudin-1 protein of Reference Test 3.

### [Description of Embodiments]

### (Agent for enhancing skin barrier)

An agent for enhancing the skin barrier of the present invention contains at least one of a monosaccharide and a sugar alcohol, and has a pH of 4.0 to 7.0. A form of the agent for enhancing the skin barrier is not particularly limited as long as it is a form that enables pH measurement. Specific examples thereof include, a liquid form, a gel form, a sheet form, a sol form, a cream form, an emulsion form, and the like, but are not limited thereto.

The agent for enhancing the skin barrier of the present invention contains at least one of a monosaccharide and a sugar alcohol. By incorporating at least one of a monosaccharide and a sugar alcohol, it is possible to promote the production of Filaggrin and Claudin-1 in cells and to enhance skin barrier functions. The agent for enhancing the skin barrier aims to enhance or restore inherent skin barrier functions of the skin, such as a function of retaining a water content of the skin for moisturization, and a function of preventing invasion of microorganisms and the like into cells.

A content of at least one of a monosaccharide and a sugar alcohol in the agent for enhancing the skin barrier is preferably 1 to 5000 mM, is more preferably 6 to 3000 mM, is even more preferably 6 to 1000 mM, and is particularly preferably 6 to 165 mM. When a content of at least one of a monosaccharide and a sugar alcohol is the above-mentioned lower limit value or more, the agent for enhancing the skin barrier promotes the production of Filaggrin and Claudin-1, and the skin barrier functions are easily enhanced or restored. When a content of at least one of a monosaccharide and a sugar alcohol is the above-mentioned upper limit value or less, at least one of a monosaccharide and a sugar alcohol is homogeneously dispersed in the agent for enhancing the skin barrier, and thereby the production of Filaggrin and Claudin-1 is sufficiently promoted, and the skin barrier functions are easily and sufficiently enhanced or restored.

### <Monosaccharide>

Examples of monosaccharides include monosaccharides such as glucose, fructose, mannose, arabinose, gulose, xylose, lyxose, erythrose, threose, galactose, and sorbose, but are not limited thereto. Among these, the agent for enhancing the skin barrier preferably contains glucose as a monosaccharide.

### <Sugar alcohol>

Examples of sugar alcohols include mannitol, sorbitol, xylitol, erythritol, maltitol, lactitol, and the like, but are not limited thereto. Among these, the agent for enhancing the skin barrier preferably contains mannitol as a sugar alcohol.

### <PH of agent for enhancing skin barrier>

A pH of the agent for enhancing the skin barrier of the present invention is 4.0 to 7.0, and is preferably 5.0 to 7.0. When a pH is the above-mentioned lower limit value or more, damage to the skin tissue and cells due to the agent for enhancing the skin barrier is reduced, and transcription and expression of Filaggrin mRNA and Claudin-1 mRNA are promoted, and thereby the skin barrier functions are easily enhanced or restored. When a pH is the above-mentioned upper limit value or less, the production of Filaggrin and Claudin-1 is sufficiently promoted, and thereby the skin barrier functions are sufficiently enhanced or restored.

In addition, in the present specification, a pH is a value measured at 25°C using a pH meter ("F-53" manufactured by HORIBA, Ltd.).

A pH of the agent for enhancing the skin barrier can be adjusted by a buffer solution.

A buffer solution is not particularly limited as long as it maintains a pH of the agent for enhancing the skin barrier at 4.0 to 7.0. Examples of buffer solutions include buffer solutions such as a phosphate buffer solution, a citrate buffer solution, a citrate-phosphate buffer solution, a maleate buffer solution, a malate buffer solution, a succinate buffer solution, a metaphosphate buffer solution, a sorbate buffer solution, a carbonate buffer solution, a tris-hydroxymethylaminomethane-HCl buffer solution (Tris-HCl buffer solution), a 2-morpholinoethanesulfonic acid buffer solution (MES buffer solution), a N-tris(hydroxymethyl)methyl-2-aminoethanesulfonic acid buffer solution (TES buffer solution), an acetate buffer solution, a 3-morpholinopropanesulfonic acid buffer solution (MOPS buffer solution), and a 4-(2-hydroxyethyl)-1-piperazineethanesulfonic acid buffer solution (HEPES buffer solution); amino acid-based buffer solutions such as a glycine-hydrochloric acid buffer solution, a glycine-NaOH buffer solution, a glycylglycine-NaOH buffer solution, and a glycylglycine-KOH buffer solution; borate-based buffer solutions such as a Tris-borate buffer solution, a borate-NaOH buffer solution, and a borate buffer solution; an imidazole buffer solution; and the like, but examples are not limited thereto. Among these, a phosphate buffer solution, a citrate buffer solution, a malate buffer solution, a succinate buffer solution, and a metaphosphate buffer solution are preferable. Adjustment of a pH of a buffer solution can be appropriately performed using an acidic substance such as hydrochloric acid and acetic acid or a basic substance such as sodium hydroxide or ammonia.

A pH of the agent for enhancing the skin barrier may be adjusted to 4.0 to 7.0 by adding an acidic substance such as phosphoric acid, acetic acid, and amino acid to the agent for enhancing the skin barrier, without adding the above-mentioned buffer solutions to the agent for enhancing the skin barrier.

In a case where a pH of the agent for enhancing the skin barrier is adjusted by the buffer solution, a concentration of the buffer solution in the agent for enhancing the skin barrier is preferably 6 to 165 mM. When a concentration of the buffer solution is the above-mentioned lower limit value or more, a pH of the agent for enhancing the skin barrier is easily adjusted. When a concentration of the buffer solution is the above-mentioned upper limit value or less, the agent for enhancing the skin barrier sufficiently promotes the production of Filaggrin and Claudin-1, and thereby the skin barrier functions are easily and sufficiently enhanced or restored.

### <Optional components>

The agent for enhancing the skin barrier can contain optional components other than at least one of a monosaccharide and a sugar alcohol as necessary as long as the effects of the present invention are not impaired.

Examples of optional components include excipients, thickeners, carriers, fragrances, colorants, and the like. These optional components may be used alone or may be used in combination of two or more kinds thereof. The optional components will be specifically described in the section of "(Medicinal composition)" to be described later.

The present invention relates to use of at least one of a monosaccharide and a sugar alcohol for the agent for enhancing the skin barrier, the use including administering at least one of a monosaccharide and a sugar alcohol at a pH of 4.0 to 7.0.

In other words, the present invention relates to use of at least one of a monosaccharide and a sugar alcohol, and a pH adjuster as an optional component for producing the agent for enhancing the skin barrier, the use including administering at least one of a monosaccharide and a sugar alcohol at a pH of 4.0 to 7.0.

### (Medicinal composition)

A medicinal composition of the present invention contains the agent for enhancing the skin barrier of the present invention, and has a pH of 4.0 to 7.0.

A content of at least one of a monosaccharide and a sugar alcohol in the medicinal composition can be determined in consideration of use applications, usage, and the like of the medicinal composition (the agent for enhancing the skin barrier). For example, in a case of a percutaneous absorption agent using a medicinal composition, a content thereof is preferably 100 to 5000 mM, and is more preferably 1000 to 3000 mM. In a case of an injection using a medicinal composition, a content thereof is preferably 1 to 1000 mM, and is more preferably 6 to 165 mM. When a content of at least one of a monosaccharide and a sugar alcohol is the above-mentioned lower limit value or more, the medicinal composition promotes the production of Filaggrin and Claudin-1, and the skin barrier functions are easily enhanced or restored. When a content of at least one of a monosaccharide and a sugar alcohol is the above-mentioned upper limit value or less, at least one of a monosaccharide and a sugar alcohol is homogeneously dispersed in the medicinal composition, and thereby the production of Filaggrin and Claudin-1 is sufficiently promoted, and the skin barrier functions are easily and sufficiently enhanced or restored.

A pH of the medicinal composition is 4.0 to 7.0, and is preferably 5.0 to 7.0. When a pH is the above-mentioned lower limit value or more, expression of mRNA encoding Filaggrin and mRNA encoding Claudin-1 is promoted, and thereby the skin barrier functions are easily enhanced or restored. When a pH is the above-mentioned upper limit value or less, damage to the skin tissue by administration of the medicinal composition is unlikely to occur, and thereby production of Filaggrin and Claudin-1 is sufficiently promoted, and the skin barrier functions are easily and sufficiently enhanced or restored.

Examples of use applications of the medicinal composition include therapeutic agents for autoimmune diseases such as atopic dermatitis, asthmatic bronchitis, rheumatoid arthritis, and collagenosis; anti-inflammatory drugs for diseases such as organ inflammation such as nephrotic syndrome, ulcerative colitis, hepatitis, pancreatitis, thyroiditis, colds, stomatitis, gingivitis, and periodontitis; wound healing drugs for wounds such as burns, wounds, pressure ulcers, acne, heat rash, and chilblains; therapeutic agents for genetic disorders such as xeroderma pigmentosum and ichthyosis; and the like. Among these, particularly, when the medicinal composition of the present invention is applied to anti-inflammatory agents and wound healing drugs, the effects of the present invention are significant.

A dosage form of the medicinal composition is not particularly limited as long as a pH can be measured. Specific examples of dosage forms include oral agents such as liquid agents, emulsion agents, suspension agents, and syrup agents; percutaneous absorption agents such as ointments, lotions, creams, adhesive patches, and aerosols; and parenteral agents such as injections, eye drops, and suppositories. Among these, the effects of the present invention are prominently exhibited in percutaneous absorption agents such as ointments. Regarding injections, from the viewpoint of stability, water may be removed by freeze-drying treatment after a vial or the like is filled with a liquid agent. In a case where water is removed, a liquid agent may be prepared by dispersing a lyophilizate in physiological saline or the like immediately before use.

The usage of the medicinal composition is appropriately determined according to the type of disease and the like, for example. In a case where a disease is a skin wound such as atopic dermatitis, incised wound, or scratch, examples of usage include a method of directly applying the medicinal composition, which is a percutaneous absorption agent, to an affected area, and the like.

In a case where a disease is in the oral cavity or the gastrointestinal tract, examples of usage include a method of orally administering the medicinal composition as an oral agent, a method of injecting the medicinal composition as an injection into an affected tissue, and the like. Mucosal regeneration may be promoted by washing an affected area with the medicinal composition and applying the medicinal composition to the affected area under an upper endoscopy. Accordingly, a healthy mucous membrane can be formed, natural healing can be gradually increased, and mucosal invasion can be prevented. In addition, by applying the medicinal composition to the gastric mucous membrane, regeneration of the gastric mucous membrane can be promoted, and thereby barrier functions of epithelial tissue can be enhanced or restored.

In a case where a disease is a disease of the nasal- or ocular-mucous membrane part, such as hay fever, examples of usage include a method of applying the medicinal composition to an affected area. When the medicinal composition is applied to the nasal- or ocular-mucous membrane part, the nasal- or ocular-mucous membrane part is repaired, and a strong barrier is formed in a mucosal layer. For this reason, even when a large number of allergens adhere to the nasal- or ocular-mucous membrane part, allergic reactions can be suppressed, and it is possible to eliminate the occurrence of nasal congestion and large amounts of secretion of nasal discharge or tears.

In a case where a disease is inflammation (cold) of the upper respiratory mucous membrane due to a virus such as colds or bacterial infection, examples of usage include a method of instilling or inhaling the medicinal composition. Accordingly, the inflammation of the upper respiratory mucous membrane can be suppressed, the upper respiratory mucous membrane can be repaired and regenerated to suppress infection progression, and the activation of an innate immune reaction can be promoted.

A dosage amount of the medicinal composition is appropriately determined according to the age and body weight of a patient or subject, the type and degree of a disease, an administration method, and the like.

The medicinal composition can contain optional components other than the agent for enhancing the skin barrier of the present invention (hereinafter, also referred to as "optional components of the medicinal product") as necessary as long as the effects of the present invention are not impaired.

Examples of optional components of the medicinal product include pharmaceutically acceptable salts, excipients, thickeners, carriers, fragrances, colorants, and the like. These optional components of the medicinal product may be used alone or may be used in combination of two or more kinds thereof.

Examples of pharmaceutically acceptable salts include generally used nontoxic salts, that is, acid addition salts and salts with various bases. Specific examples thereof include mineral acid salts such as hydrochloric acid salts, nitric acid salts, and sulfuric acid salts; organic acid salts such as acetic acid salts, citric acid salts, fumaric acid salts, and tartaric acid salts; sulfonic acid salts such as methane sulfonic acid salts and p-toluene sulfonic acid salts; amino acid salts such as alanine salts, leucine salts, and glutamic acid salts; inorganic base salts such as alkali metal salts (such as sodium salts and potassium salts) and alkaline earth metal salts (such as magnesium salts and calcium salts); organic amine salts such as triethylamine salts, pyridine salts, picoline salts, ethanolamine salts, triethanolamine salts, dicyclohexylamine salts, and N,N'-dibenzylethylenediamine salts; and the like. These salts may be used alone or may be used in combination of two or more kinds thereof. When such salts are contained, crystallization is easy.

An excipient can be appropriately selected depending on the dosage form of the medicinal composition. Specific examples thereof include water such as distilled water, ion exchange water, and pure water; lower alcohols having 1 to 6 carbon atoms such as methanol and ethanol; liquid excipients such as glycerin; solid excipients such as lactose, starch, dextrin, and sucrose; and the like. These excipients may be used alone or may be used in combination of two or more kinds thereof.

A thickener can be appropriately selected depending on the dosage form of the medicinal composition. Specific examples thereof include gelatin, xanthan gum, carrageenan, and the like. These thickeners may be used alone or may be used in combination of two or more kinds thereof.

The present invention relates to use of at least one of a monosaccharide and a sugar alcohol for the medicinal composition in the treatment of skin diseases, the use including administering at least one of a monosaccharide and a sugar alcohol at a pH of 4.0 to 7.0.

In other words, the present invention relates to use of at least one of a monosaccharide and a sugar alcohol, and a pH adjuster as an optional component for producing the medicinal composition in the treatment of skin diseases, the use including administering at least one of a monosaccharide and a sugar alcohol at a pH of 4.0 to 7.0.

The medicinal composition of the present invention can be used in a method of enhancing skin barrier functions. The method of enhancing skin barrier functions includes administering an active ingredient amount or more of at least one of a monosaccharide and a sugar alcohol at a pH of 4.0 to 7.0.

### (Medicated cosmetic)

A medicated cosmetic of the present invention contains the agent for enhancing the skin barrier of the present invention, and has a pH of 4.0 to 7.0. The medicated cosmetic of the present invention is, for example, lotions, creams, emulsions, foundations, and the like, and is for preventing skin diseases and moisturizing the skin. Medicated cosmetics are classified as quasi-drugs as defined in the Pharmaceutical Affairs Law of Japan.

A content of the agent for enhancing the skin barrier in the medicated cosmetic can be determined in consideration of use applications, administration forms, and the like of the medicated cosmetic. For example, a content thereof is such that a concentration of at least one of a monosaccharide and a sugar alcohol in the medicated cosmetic is preferably 1 to 1000 mM, and is more preferably 6 to 165 mM. When a concentration of at least one of a monosaccharide and a sugar alcohol is within the above-mentioned range, the production of Filaggrin and Claudin-1 is promoted, the skin barrier functions are sufficiently enhanced or restored, and thereby skin tissue repair is further promoted.

A pH of the medicated cosmetic is 4.0 to 7.0, and is preferably 5.0 to 7.0. When a pH is the above-mentioned lower limit value or more, expression of Filaggrin mRNA and Claudin-1 mRNA is promoted, and thereby the skin barrier functions are easily enhanced or restored. When a pH is the above-mentioned upper limit value or less, damage to the skin tissue by administration of the medicinal composition is unlikely to occur, and thereby production of Filaggrin and Claudin-1 is sufficiently promoted, and the skin barrier functions are easily and sufficiently enhanced or restored.

A form of the medicated cosmetic is not particularly limited as long as a pH can be measured. As specific examples, medicated cosmetics in a liquid form, a gel form, a sheet form, and the like are exemplified.

A use method of the medicated cosmetic is appropriately determined according to the purpose of beauty care, for example. In a case where the medicated cosmetic is for beauty care and moisturization of skin, examples of use methods include a method of directly applying medicated cosmetics in a liquid form, an emulsion form, a gel form, and a sheet form to the skin; and the like.

In a case where the purpose is beauty care of the oral cavity, examples of use methods include a method of orally applying medicated cosmetics in a liquid form, a gel form, and the like; a method of injecting a medicated cosmetic in a liquid form as an injection; and the like.

The medicated cosmetic can contain optional components other than the agent for enhancing skin barrier of the present invention (hereinafter, also referred to as "optional components of the cosmetic") as necessary as long as the effects of the present invention are not impaired.

Examples of optional components of the cosmetic include oil agents such as liquid paraffin, ceresin, Japan wax, lanolin, vaseline, cetanol, squalene, jojoba oil, stearic acid, palmitic acid, lauryl alcohol, stearyl alcohol, cetyl alcohol, beeswax, methylpolysiloxane, and dimethylcyclopolysiloxane; moisturizing agents such as propanol, glycol, propylene glycol, hyaluronic acid, collagen, polyethylene glycol, cholesteryl hydroxystearate, glycerin, and sorbitol; various surfactants; emulsifiers; excipients; thickeners; pH adjusters; antioxidants; colorants; fragrances; ultraviolet absorbers; and the like. These optional components of the cosmetic may be used alone or may be used in combination of two or more kinds thereof.

The present invention relates to use of at least one of a monosaccharide and a sugar alcohol for the medicated cosmetic in moisturization or beauty care of skin, the use including administering at least one of a monosaccharide and a sugar alcohol at a pH of 4.0 to 7.0.

In other words, the present invention relates to combination use of at least one of a monosaccharide and a sugar alcohol, and a pH adjuster as an optional component for producing the medicated cosmetic in moisturization or beauty care of skin, the use including administering at least one of a monosaccharide and a sugar alcohol at a pH of 4.0 to 7.0.

### (Beauty care method)

A beauty care method of the present invention includes a first step of applying the above-described medicated cosmetic of the present invention to skin or a mucous membrane. An application amount of the medicated cosmetic is appropriately determined according to the age, weight, and condition of skin of a patient or subject, an application method, and the like.

The beauty care method of the present invention preferably includes, after the first step, a second step of returning a pH of the skin or the mucous membrane to a basic region of greater than 7.0. A time from the first step to the second step is preferably about 15 to 30 minutes. When an interval between the first step and the second step is about 15 to 30 minutes, a pH easily returns to a basic region which is the original pH of the skin or the mucous membrane. Examples of methods of returning a pH to a basic region include a method of washing away with running water; a method of removing a medicated cosmetic by wiping it off; a method of applying a cream or the like, which contains a neutralizing agent, on a medicated cosmetic to skin or a mucous membrane; and the like. These methods may be used alone or may be used in combination of two or more kinds thereof.

In the beauty care method of the present invention, it is preferable to repeatedly perform the first step and the second step described above.

The beauty care method of the present invention includes administering an active ingredient amount or more of at least one of a monosaccharide and a sugar alcohol at a pH of 4.0 to 7.0. When administering at least one of a monosaccharide and a sugar alcohol, at least one of a monosaccharide and a sugar alcohol may be applied to the skin or mucous membrane of a patient or subject.

### (Effects)

As described above, the agent for enhancing the skin barrier of the present invention contains at least one of a monosaccharide and a sugar alcohol, has a pH of 4.0 to 7.0, and thereby can promote the production of Filaggrin and Claudin-1 in cells, and can promote transcription and expression of Filaggrin mRNA and Claudin-1 mRNA. Accordingly, the skin barrier functions are sufficiently enhanced or restored by the agent for enhancing the skin barrier.

Although the embodiments of the present invention have been described above in detail, the present invention is not limited to such specific embodiments.

### [Examples]

Hereinafter, the present invention will be described in detail by showing examples, but the present invention is not limited by the following description.

### (Examples 1 to 7 and Comparative Examples 1 to 7)

D-glucose (product number: G5767, manufactured by Sigma-Aldrich) or D-mannitol (product number: M9647, manufactured by Sigma-Aldrich), and hydrochloric acid (HCl) for adjusting a pH of a medium (product number: 080-0106, manufactured by Wako Pure Chemical Industries, Ltd.) were added to a HuMedia-KG2 medium (manufactured by KURABO INDUSTRIES LTD.) so that a concentration became as shown in Table 1, and thereby a medium (hereinafter, will be described as the "test medium") containing an agent for enhancing the skin barrier of which a pH was adjusted as shown in Table 1 was prepared. ApH is a value measured at 25°C using a pH meter ("F-53" manufactured by HORIBA, Ltd.).

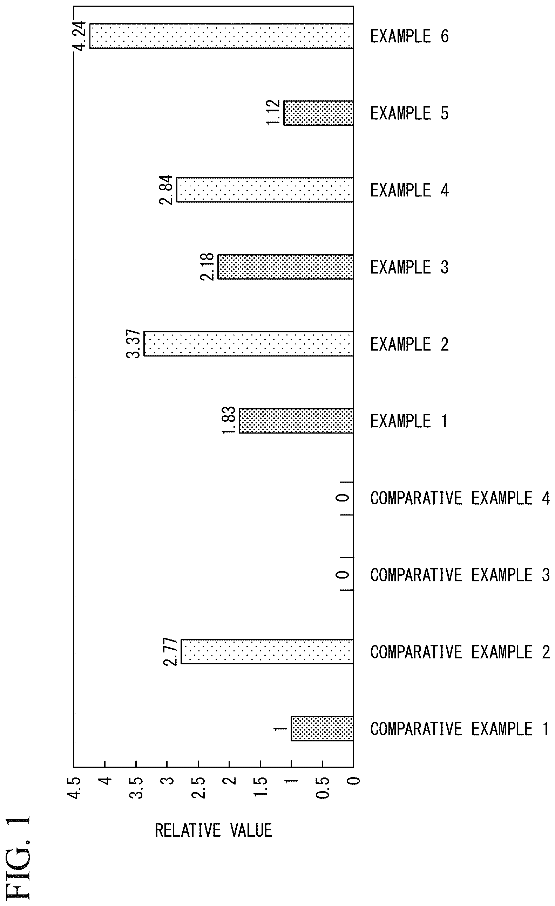

Using the test media of Examples 1 to 7 and Comparative Examples 1 to 4 and 7, analysis of an expression level of a Filaggrin protein described below, and analysis of respective expression levels of mRNA of a Filaggrin protein and mRNA of a Claudin-1 protein were performed. The results are shown in Figs. 1 to 4.

### <Analysis 1 of expression level of Filaggrin protein>

Using a HuMedia-KG2 medium (pH 7.6, manufactured by KURABO INDUSTRIES LTD.), normal human skin keratinocytes (normal skin keratinocytes in primary culture which were derived from a single donor and were manufactured by Takara Bio Inc.) were seeded in a 6-well microplate at a cell density of 1,000,000 cells/well, and cultured at 37°C for 24 hours (preculture). After the preculture, a culture solution was removed by suction, the medium was replaced with the test media of Examples 1 to 6 and Comparative Examples 1 to 4, and culture was performed at 37°C for 30 minutes (main culture). After the main culture, the culture supernatant was removed by suction, the medium was replaced with HuMedia-KG2 medium having a pH of 7.6, and culture was performed at 37°C for 3 hours (post-culture). After the post-culture, the medium was removed, and proteins were extracted from the cultured cells. The extracted proteins were separated by SDS-PAGE. Thereafter, the extracted proteins were transferred to a nitrocellulose membrane, and an expression level of the Filaggrin protein in the cultured cells was analyzed by western blot method using a Filaggrin antibody derived from a mouse. In addition, as an endogenous control, an expression level of a β-actin protein in the same sample was analyzed. A concentration of respective protein bands detected was measured with a densitometer, and expression levels of the Filaggrin protein and β-actin protein were analyzed. The expression level of the Filaggrin protein was evaluated with the expression level of the β-actin protein in the same sample as 1, and the expression level of the Filaggrin protein as a relative value (a relative expression level). The results are shown in Fig. 1.

### <Analysis of expression levels of Filaggrin mRNA and Claudin-1 mRNA>

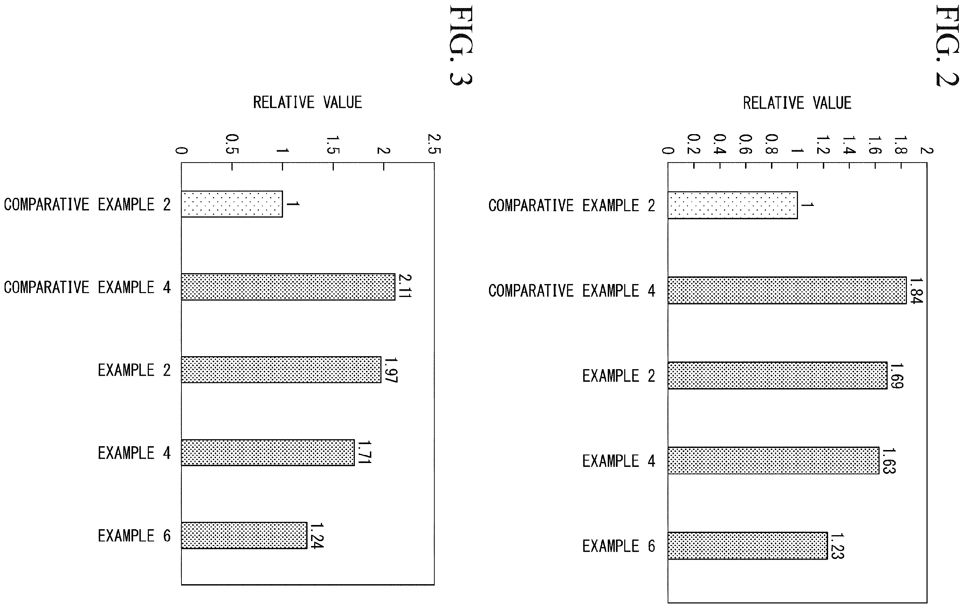

Preculture, main culture, and post-culture were performed in the same manner as in "<Analysis 1 of expression level of Filaggrin protein>" except that the test medium was changed to the test media of Examples 2, 4, and 6 and Comparative Examples 2 and 4. After the post-culture, the medium was removed, and total RNAs were extracted from the cultured cells. Using RT-PCR method, mRNAs of Filaggrin and Claudin-1 were amplified from the extracted RNAs, and expression levels of each mRNA were analyzed. The expression levels of the Filaggrin mRNA and the Claudin-1 mRNA were evaluated with the expression level of each mRNA of Comparative Example 2 as 1, and the expression level of each mRNA of Examples 2, 4, and 6 and Comparative Example 4 as a relative value (a relative expression level). The results are shown in Figs. 2 and 3.

### <Analysis 2 of expression level of Filaggrin protein>

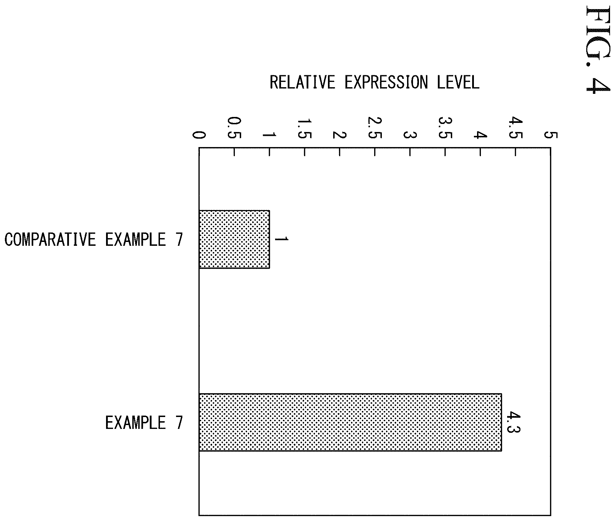

Analysis of expression levels of a Filaggrin protein of Example 7 and Comparative Example 7 was performed in the same manner as in "<Analysis 1 of expression level of Filaggrin protein>" except that the test media of Example 7 and Comparative Example 7 were used. The expression level of the Filaggrin protein of Example 7 was evaluated with the expression level of the Filaggrin protein of Comparative Example 7 as 1, and a relative value (a relative expression level) of the expression level of the Filaggrin protein of Example 7 with respect to the expression level of the Filaggrin protein of Comparative Example 7. The results are shown in Fig. 4.

**[Table 1]**

| | Example 1 | Example 2 | Example 3 | Example 4 | Example 5 | Example 6 | Example 7 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|---|---|---|---|
| Concentration of D-glucose (mM) | 6 | 110 | 6 | 110 | 6 | 110 | 0 | 6 | 110 | 6 | 110 | 165 | 55 | 0 |
| Concentration of D-mannitol (mM) | 0 | 0 | 0 | 0 | 0 | 0 | 110 | 0 | 0 | 0 | 0 | 0 | 0 | 110 |
| pH | 5.0 | 5.0 | 6.0 | 6.0 | 7.0 | 7.0 | 5.7 | 7.6 | 7.6 | 3.0 | 3.0 | 7.6 | 7.6 | 7.6 |

As shown in Table 1 and Fig. 1, in Examples 1 to 6 to which the present invention was applied, the expression levels of the Filaggrin protein increased. In particular, in Examples 2, 4, and 6 which contain 110 mM of D-glucose, the expression levels of the Filaggrin protein remarkably increased.

As shown in Table 1 and Figs. 2 and 3, in Examples 2, 4, and 6 to which the present invention was applied, the expression levels of respective mRNAs of Filaggrin and Claudin-1 increased. In Comparative Example 4 in which 110 mM of D-glucose was contained and a pH was 3.0, an increase in respective mRNAs of Filaggrin and Claudin-1 was also observed.

As shown in Table 1 and Fig. 4, in Example 7 to which the present invention was applied, the expression level of the Filaggrin protein increased 4.3-fold as compared with Comparative Example 7.

Based on the results of Examples 1 to 6 and Comparative Examples 1 to 4, it was found that production of a Filaggrin protein by cultured cells can be promoted by adjusting a glucose concentration of a test medium to 6 mM or 110 mM and adjusting a pH to 4.0 to 7.0.

Based on the results of Examples 1 to 6 and Comparative Examples 2 and 4, it was found that the expression of respective mRNAs of Filaggrin and Claudin-1 in cultured cells can be promoted by adjusting a glucose concentration of a test medium to 110 mM and adjusting a pH to 3.0 to 7.0.

Based on the results of Comparative Examples 3 and 4, it was found that the expression of mRNA of Filaggrin protein is promoted by adjusting a glucose concentration to 110 mM and a pH to 3.0, but the Filaggrin protein is not detected.

Based on the results of Example 7 and Comparative Example 7, it was found that production of a Filaggrin protein by cultured cells can be promoted by adjusting a mannitol concentration of a test medium to 110 mM and adjusting a pH to 5.7. Based on the above-described results, it was found that the application of the present invention promotes the expression of Filaggrin and Claudin-1, and thereby can enhance the skin barrier functions.

### <Cell damage test>

Normal human skin keratinocytes were cultured in the same manner as in the section of "<Analysis 1 of expression level of Filaggrin protein>." The degree of damage to the cultured cells was analyzed by an LDH method. The damage percentage of the cells cultured in each example was evaluated with a degree of damage to cells obtained by treating cells cultured in the test medium of each example with Triton X 100 as 100%.

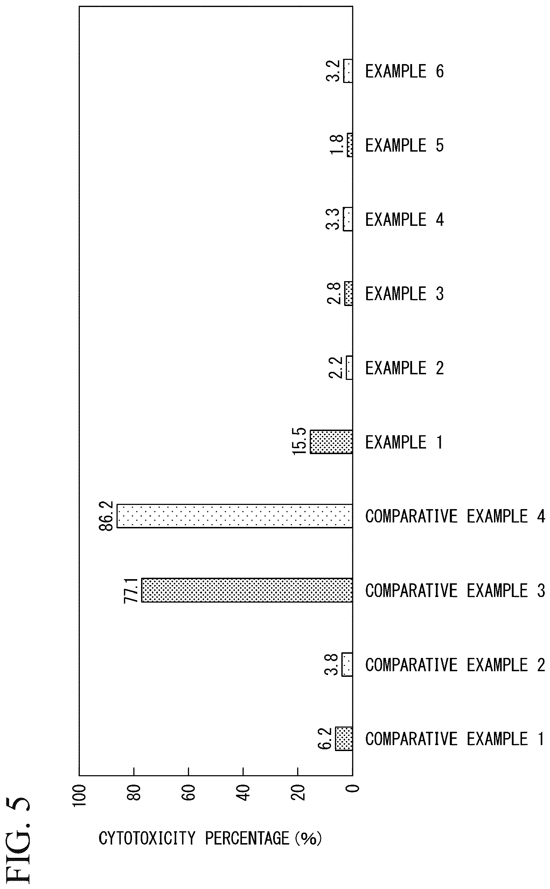

Fig. 5 is a graph showing the results of evaluating a damage percentage of cells cultured in the test media of Examples 1 to 6 and Comparative Examples 1 to 4.

As shown in Table 1 and Fig. 5, a damage percentage of the cells of Examples 1 to 6 to which the present invention was applied was lower than a damage percentage of the cells of Comparative Examples 1 and 2 in which a pH was 7.6. On the other hand, a damage percentage of the cells of Comparative Examples 3 and 4 in which a pH was 3.0 was higher than a damage percentage of the cells of Comparative Examples 1 and 2 in which a pH was 7.6.

Based on the above-described results, the occurrence of cell damage was recognized in Comparative Examples 3 and 4 in which a pH was 3.0. However, this cell damage was found to be significantly reduced by applying the present invention to a test medium in an acidic region with a pH of 5.0 or more, which is a reduction to a level lower than cell damage recognized in the cultured cells in the test media of Comparative Examples 1 and 2 in which a pH was 7.6.

In other words, it was found that by applying the present invention to a test medium having a pH of 5.0 or more, the skin barrier functions are restored and the cell damage is reduced.

### <Evaluation of mouse model with atopic dermatitis>

An aqueous solution of phosphate-buffered saline (PBS) in which a glucose concentration was prepared to 20% and a pH was adjusted to 6.0 was produced and used as an agent for enhancing the skin barrier to which the present invention was applied.

A mite antigen was intradermally administered in frequency of twice a week from Day 0 to Day 14 to the right pinna of 8-week-old female NC/Nga mice after habituation breeding. At the same time, the agent for enhancing the skin barrier of the present invention was applied to be administered once a day from Day 0 to Day 28. As Mock group, PBS in which a pH was adjusted to 7.6 was applied to be administered once a day from Day 0 to Day 28.

Pictures of the pinnae of the Mock group and the group to which the agent for enhancing the skin barrier was applied were photographed on Day 28, left and right pinnae were collected from exsanguinated and euthanized mice under isoflurane anesthesia, and neutral formalin fixation was performed. After paraffin embedding, tissue sections were produced. The sections were stained with a hematoxylin-eosin (HE) solution and immunostained using Filaggrin and Claudin-1 antibodies derived from mice. As a negative control, pinna sections of an 8-week-old female NC/Nga mouse which is a mouse bred with no treatment until Day 28 were used.

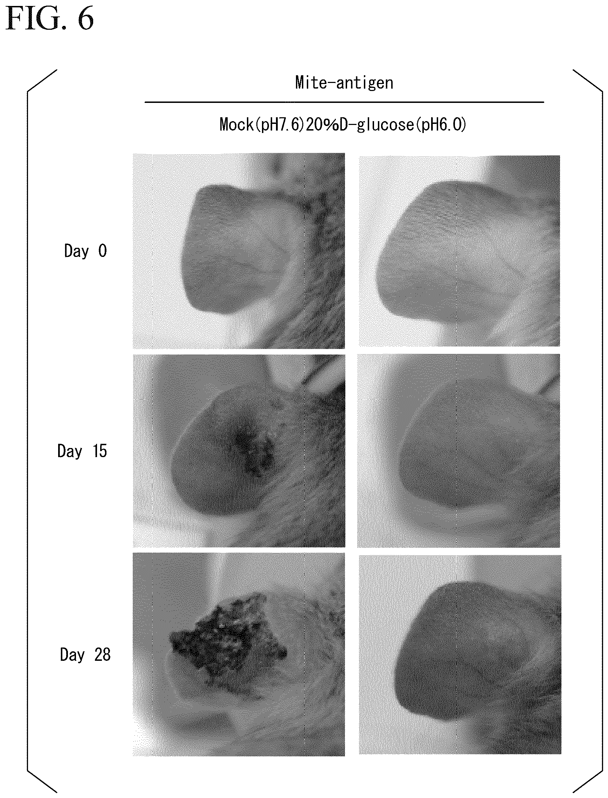

Fig. 6 shows photographs of the appearance of a mouse pinna evaluated by a mouse model with atopic dermatitis. As a result of visual observation of the pinna of the mouse, in the group (THE Mock group) in which PBS with a pH of 7.6 was applied to the pinna of the mouse sensitized with the mite antigen, a mild degree of ulceration and bleeding were recognized on Day 15, and a severe degree of bleeding and ulceration were observed on Day 28. On the other hand, in the group to which the agent for enhancing the skin barrier of the present invention was applied, slight swelling of the skin was recognized on Day 28, but no ulceration and bleeding were recognized neither on Day 15 nor Day 28.

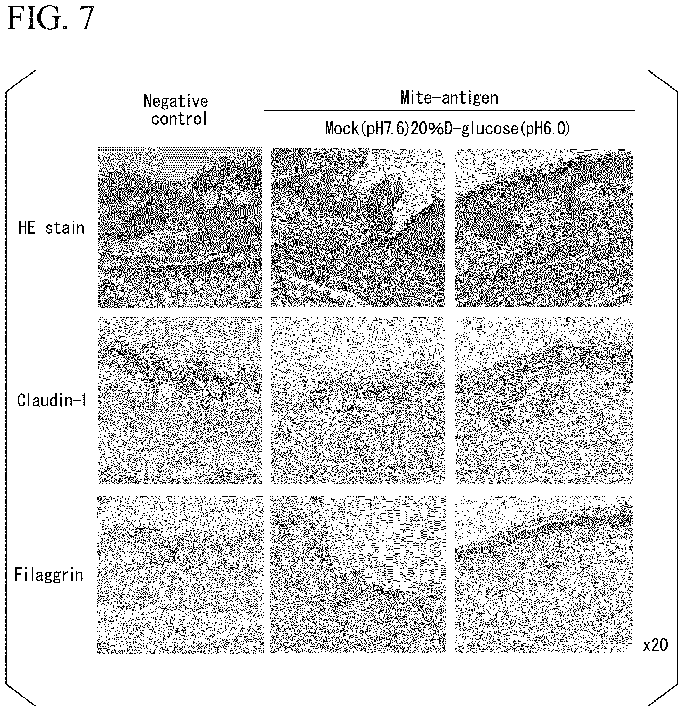

Fig. 7 shows photographs of results of staining tissue sections of the mouse pinna evaluated by the mouse model with atopic dermatitis. As shown in the HE stain results in Fig. 7, infiltration of inflammatory cells in the dermis was strongly observed in the Mock group. However, in the group to which the agent for enhancing the skin barrier of the present invention was applied, infiltration of inflammatory cells was reduced, and morphologies of the stratum corneum and the epidermis were maintained normally. As shown in the results of immunostaining in Fig. 7, in the Mock group, the continuity of expression of a Claudin-1 protein was lost, and an expression level thereof decreased. On the other hand, in the group to which the agent for enhancing the skin barrier of the present invention was applied, a stained image of strongly continued Claudin-1 protein was recognized. Similarly, in the Mock group, a decreased expression and loss of continuity of a Filaggrin protein were observed. On the other hand, in the group to which the agent for enhancing the skin barrier of the present invention was applied, a Filaggrin protein was continuously and widely expressed in the stratum granulosum.

### <Reference Test 1>

Using a HuMedia-KG2 medium (pH 7.6, manufactured by KURABO INDUSTRIES LTD.), normal human skin keratinocytes were seeded in a 6-well microplate at a cell density of 1,000,000 cells/well, and cultured at 37°C for 24 hours (preculture). After the preculture, a culture solution was removed by suction, the medium was replaced with the test media of Comparative Example 2, and main culture was performed at 37°C. The medium was removed from respective culture solutions for which a time for the main culture was set to 0, 0.5, 1.0, 2.0, 3.0, 4.0, and 5.0 hours, respectively, and total RNAs were extracted from the cultured cells. Using RT-PCR method, mRNAs of Filaggrin and Claudin-1 were amplified from the RNAs extracted from respective culture solutions, and expression levels of respective mRNAs were analyzed for respective culture solutions having different times for the main culture. The expression levels of respective mRNAs of Filaggrin and Claudin-1 were evaluated with the expression level of each mRNA when the time for the main culture was 0 hours as 1, and the expression level of each mRNA of cells of each culture time as a relative value (a relative expression level).

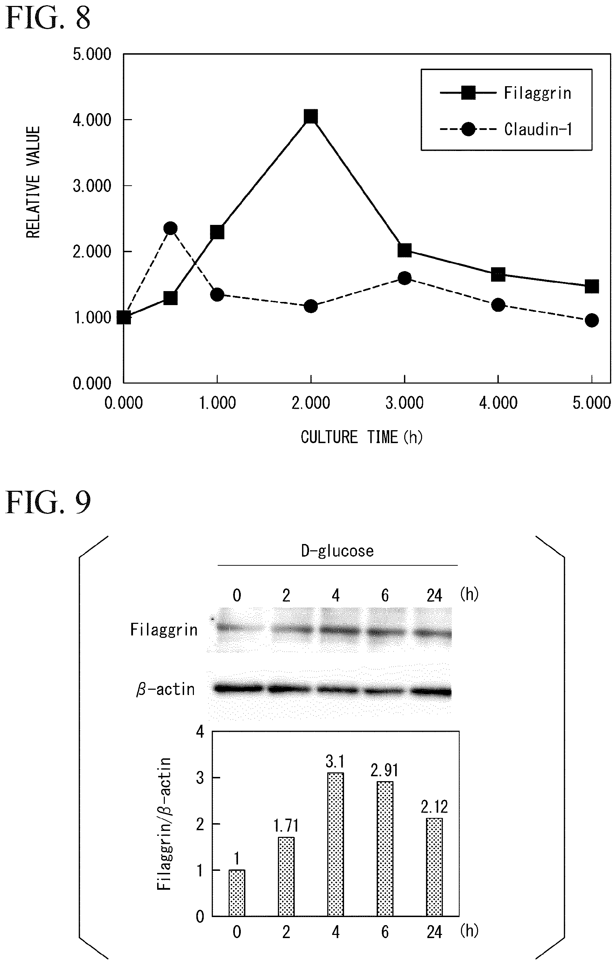

Fig. 8 is a graph showing evaluation results of mRNA expression levels of Filaggrin and Claudin-1 of Comparative Example 2 at each culture time of Reference Test 1. In Comparative Example 2, a peak of expression induction of Claudin-1 mRNA was recognized at 0.5 hours after the start of the expression induction. A peak of expression induction of Filaggrin mRNA was recognized at 2.0 hours after the start of the expression induction.

### <Reference Test 2>

An expression level of the Filaggrin protein at each culture time was evaluated in the same manner as in the section of "<Analysis 1 of expression level of Filaggrin protein>" except that the test medium of Comparative Example 5 was used, and the time for the main culture time was set to 0, 2.0, 4.0, 6.0, and 24.0 hours.

Fig. 9 is a graph showing evaluation results of an expression level of a Filaggrin protein of Reference Test 2. After two hours from the start of induction of expression, an increase in the expression level of the Filaggrin protein was recognized. An increase in the expression level of the Filaggrin protein was recognized for up to 24 hours after the start of the expression induction.

### <Reference Test 3>

An expression level of a Claudin-1 protein at each culture time was evaluated in the same manner as in the section of "<Reference Test 2>" except that the test medium of Comparative Example 6 was used, and a Claudin-1 antibody derived from mice was used.

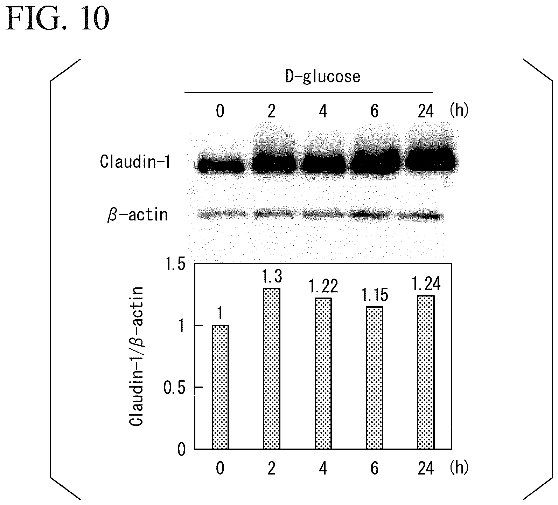

Fig. 10 is a graph showing evaluation results of an expression level of a Claudin-1 protein of Reference Test 3. After two hours from the start of induction of expression, an increase in the expression level of the Claudin-1 protein was recognized. An increase in the expression level of the Claudin-1 protein was recognized for up to 24 hours after the start of the expression induction.

Based on the results of the respective tests, gene expression of proteins relating to the skin barrier is shown to be induced by the agent for enhancing the skin barrier which contains 6 mM or 110 mM of glucose or 110 mM of mannitol and has a pH of 4.0 to 7.0. In other words, the agent for enhancing the skin barrier of the present invention was shown to be able to promote the gene expression of proteins relating to the skin barrier, thereby promoting the production of Filaggrin and Claudin-1 which are proteins relating to the skin barrier, and enhancing the skin barrier functions.

As shown in the results of Reference Tests 2 and 3, even in Comparative Examples 2, 5, and 6 in which a pH was 7.6, an increase in an expression level and a production amount of proteins relating to the skin barrier was recognized by incorporating 55 to 165 mM of glucose (Figs. 8 to 10). However, the agent for enhancing the skin barrier of the present invention can further enhance the expression of proteins relating to the skin barrier by setting a pH to 4.0 to 7.0 (Fig. 1 and Fig. 4). Accordingly, the agent for enhancing the skin barrier of the present invention can further effectively enhance the skin barrier functions than agents for enhancing skin barrier of the related art. Therefore, when the agent for enhancing the skin barrier of the present invention is applied to medicinal products and the like, significant effects such as being able to repair skin tissue more effectively can be expected.

### [Industrial Applicability]

The agent for enhancing the skin barrier, the medicinal composition, and the medicated cosmetic which are obtained by the present invention can further enhance the skin barrier functions, and can be used for moisturization or beauty care of skin, for enhancing the skin barrier functions, and the like.

## Claims

1. An agent for enhancing the skin barrier, comprising:
at least one of a monosaccharide and a sugar alcohol,
wherein a pH is 4.0 to 7.0.

2. The agent for enhancing the skin barrier according to claim 1, wherein glucose is contained as the monosaccharide.

3. The agent for enhancing the skin barrier according to claim 1, wherein mannitol is contained as the sugar alcohol.

4. The agent for enhancing the skin barrier according to any one of claims 1 to 3, wherein the pH is 5.0 to 7.0.

5. A medicinal composition comprising:
the agent for enhancing the skin barrier according to any one of claims 1 to 4,
wherein a pH is 4.0 to 7.0.

6. A medicated cosmetic comprising:
the agent for enhancing the skin barrier according to any one of claims 1 to 4,
wherein a pH is 4.0 to 7.0.

7. The medicated cosmetic according to claim 6, which is in a liquid form, a gel form, or a sheet form.

8. A beauty care method comprising a step of applying the medicated cosmetic according to claim 6 or 7 to skin or a mucous membrane.
